# EUROPÄISCHE PATENTSCHRIFT

(11) **EP 1 101 139 B1**
(45) Veröffentlichungstag und Bekanntmachung des Hinweises auf die Patenterteilung: **15.12.2004**
(21) Anmeldenummer: 99938378.9
(22) Anmeldetag: 30.07.1999
(51) Int. Cl.: G02B 7/00, A61B 19/00

(54) **OPERATIONSMIKROSKOP-STATIV FÜR X-Y-VERSCHIEBUNG**
OPERATING MICROSCOPE STAND FOR X-Y DISPLACEMENT
STATIF POUR MICROSCOPE POUR OPERATIONS POUR DEPLACEMENT X-Y

(30) Priorität: 31.07.1998 CH 162098
(43) Veröffentlichungstag der Anmeldung: 23.05.2001
(73) Patentinhaber: Leica Microsystems (Schweiz) AG, 9435 Heerbrugg (CH)
(72) Erfinder: PENSEL, Jürgen, CH-9450 Altstätten (CH)
(74) Vertreter: Rosenich, Paul, Dipl.-Ing.
(86) Internationale Anmeldenummer: PCT/EP1999/005448
(87) Internationale Veröffentlichungsnummer: WO 2000/008508

(56) Entgegenhaltungen:
- EP-A- 0 554 711
- EP-A- 0 677 278
- DE-A- 1 901 180
- DE-A- 2 161 396
- DE-A- 4 032 207
- DE-A- 4 320 443
- DE-A- 4 342 717
- DE-A- 4 416 178
- US-A- 5 345 538
- US-A- 5 661 598
- PATENT ABSTRACTS OF JAPAN vol. 1995, no. 06, 31. Juli 1995 (1995-07-31) -& JP 07 072394 A (OLYMPUS OPTICAL CO LTD), 17. März 1995 (1995-03-17)

## Beschreibung

Manche herkömmliche Stative - z.B. solche für Operationsmikroskope für die Ophthalmologie - tragen an ihrem freien Ende, zwischen dem Mikroskop und dem vertikalen Stativträger, eine X-Y-Verschiebeeinheit für das Mikroskop. Diese Verschiebeeinheit dient dazu, das Mikroskop im Millimeterbereich in X-Y-Richtung zu positionieren. Eine solche Anordnung der X-Y-Verschiebeeinheit ist in der Regel für einen Operateur störend, da sowohl die Sicht als auch die Bewegungsfreiheit eingeschränkt werden. Zudem erhöht die X-Y-Verschiebeeinheit das Gewicht am Auslegerarm deutlich und muß in der Regel über ein entsprechendes Ausgleichsgewicht kompensiert oder durch einen entsprechend großen Stativfuß abgestützt werden. In der Folge davon müssen damit auch die gesamte Tragarmkonstruktion des Stativträgers und gegebenenfalls auch die gesamte Stativfusskonstruktion stärker bemessen und vor allem großflächiger dimensioniert sein.

Der Erfindung liegt somit die Aufgabe zugrunde, einen Stativaufbau zu finden, bei dem die X-Y-Verstellfunktion erhalten bleibt, eine deutliche Gewichtserhöhung der Tragarmkonstruktion und die sich daraus ergebenden weiteren Nachteile jedoch vermieden werden.

Die Lösung der Aufgabe liegt in der erfindungsgemäßen Verlagerung der X-Y-Verschiebeeinheit an den vertikalen Stativständer, so daß die X-Y-Positioniereinheit nicht nur das Mikroskop, sondern auch wenigstens einen Teil des horizontalen Stativträgers bewegt.

Erfindungsgemäß ist die X-Y-Verschiebeeinheit nicht zwingend auf schlittenförmige Verschiebebahnen eingeschränkt. Sie kann z.B. auch aus wenigstens zwei - motorisch angetriebenen Gelenken des horizontalen Stativträgers bestehen, deren aufeinander abgestimmte Schwenkbewegungen eine beliebige Lagerveränderung des Mikroskops in einer X-Y-Ebene erlauben. Der Vorteil solcher motorischer Antriebe - mit integrierten Inkrementalgebern und entsprechender Steuerung liegt im geringen Gewicht derselben und in einem rel. günstigeren Preis, gemessen an x-y-Linearverschiebeeinheiten.

Erfindungsgemäß wird der horizontale Stativträger dabei - wie bisher bei ophthalmologischen Mikroskopen in der Regel nicht üblich - über den vertikalen Stativständer hinaus verlängert und dort mit einem Gegengewicht versehen, so daß die X-Y-Verstelleinrichtung unterhalb bzw. zumindest in der Nähe des Schwerpunktes dieses verlängerten horizontalen Trägers angreift.

Eine weitere verbesserte Ausführungsform koppelt den horizontalen Stativträger mit dem Träger für das Ausgleichsgewicht so, daß das Schwenken des Mikroskops zu einem Schwenken des Ausgleichsgewichtes in die Ausgleichsrichtung zum Zwecke der dynamischen Gewichtsbalancierung führt.

Die Verlagerung der X-Y-Verstelleinrichtung kann auch unabhängig von der Verschwenkbarkeit des verlängerten Horizontalträgers angewendet werden.

Die Lösung der oben angegebenen Aufgabe ist in der Kombination der Merkmale des Anspruches 1 unter Schutz gestellt. Durch die Erfindung gelingt es, das Gewicht und das Volumen am Lastarm des Stativs zu reduzieren und derart die Stativkonstruktion insgesamt zu verkleinern. Allfällige Ausgleichseinrichtungen und gegebenenfalls auch der Stativfuß werden dimensionell verkleinert und leichter.

Außerdem entfällt die Notwendigkeit der Zuführung von elektrischen Steuerleitungen über den horizontalen Stativträgerarm für die Antriebe in der X-Y-Verschiebeeinheit. Der Gesamtaufbau wird dadurch kompakter und leichter.

Erfindungsgemäß ist die X-Y-Verschiebeeinheit etwa im axialen Bereich des Stativständers angeordnet.

In jedem Fall ist die Anordnung der X-Y-Verschiebeeinheit im axialen Bereich des Stativständers von Vorteil, da die Verschiebeeinheit selbst an diesem Ort nicht ausbalanciert werden muß.

Gemäß einer - auch unabhängig anwendbaren - Ausgestaltung der Erfindung mit einem zweiteiligen Stativträger sind die beiden Teile des Trägers zueinander schwenkbar, wobei die Schwenkbarkeit getriebegekoppelt ist. Das heißt, beim Schwenken der Last in eine bestimmte Richtung schwenkt das Ausgleichsgewicht in die entgegengesetzte Richtung, um derart die räumliche Gewichtsverlagerung wieder zu kompensieren.

Da der Chirurg bei der praktischen Anwendung die X-Achse quer und die Y-Achse koaxial zur Längsachse des Patienten bevorzugt, wird bei einem Ausführungsbeispiel die allfällige Rotation des horizontalen Stativträgers um die Achse der Verschiebeeinheit vorgenommen, so daß das Koordinatensystem davon nicht berührt ist.

Bei einer konstruktiven Ausführung über eine fixe Achse im Stativständer oder bei Aufteilung der X-Y-Verschiebeeinheit auf mindestens zwei, im horizontalen Stativträger-Arm befindliche Gelenke, werden die entsprechenden Verschiebevektoren rechnerisch bestimmt und automatisch, rechnergestützt eingestellt.

Weitere Merkmale der Erfindung und Ausgestaltungsvarianten sind in den abhängigen Ansprüchen beschrieben bzw. unter Schutz gestellt. Weitere Merkmale und schutzfähige Details ergeben sich aus den Figuren und der Figurenbeschreibung.

Die Figurenbeschreibung ist übergreifend. Gleiche Bezugszeichen bedeuten gleiche Bauteile, Bezugszeichen mit unterschiedlichen Indizes bedeuten Bauteile mit gleichen Aufgaben, jedoch unterschiedlichen konstruktiven Ausgestaltungen.

Es zeigen dabei schematisch:
- Fig. 1: einen Aufbau gemäß Stand der Technik mit einer X-Y-Verschiebeeinheit unmittelbar oberhalb des Mikroskops;
- Fig. 4: einen Aufbau mit einem zweiarmigen Stativträger;
- Fig. 5: einen Aufbau vergleichbar mit Fig. 4 alternativ zur Erfindung, jedoch mit Verschwenkbarkeit der beiden Stativträgerteile;

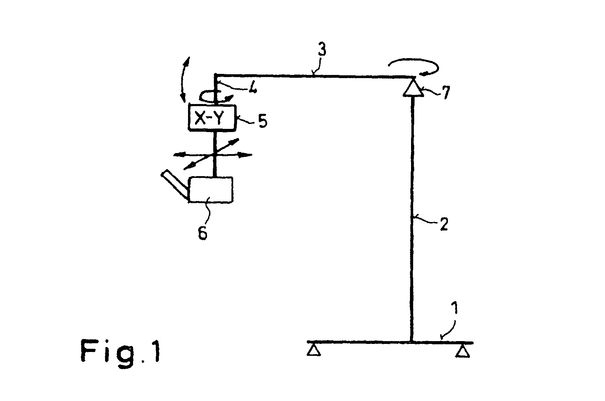

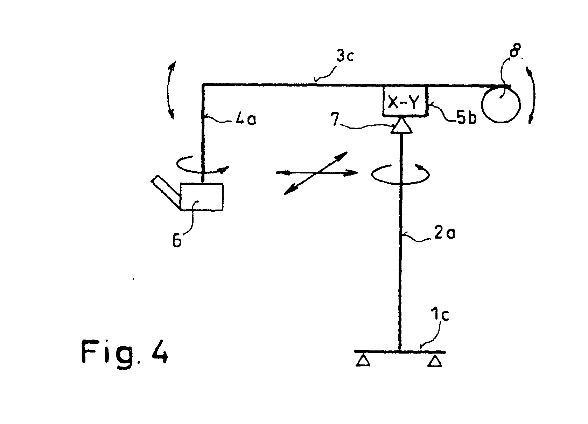

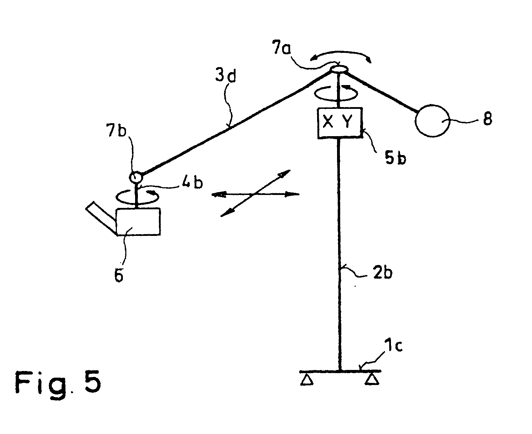

Der erste und erfindungswesentliche Schritt liegt ersichtlich im Unterschied zwischen der Fig. 1 und der Fig. 4. Gemäß Stand der Technik war die X-Y-Verschiebeeinheit 5 unmittelbar oberhalb des Mikroskops 6 an einem Mikroskophalter 4 befestigt bzw. hat den Mikroskophalter 4 unterteilt, so daß sein unterer Teil von der X-Y-Verschiebeeinheit 5 getragen wurde und das Mikroskop 6 aufnahm, während der obere Teil die X-Y-Verschiebeeinheit 5 trug. Ein Verfahren der X-Y-Verschiebeeinheit bewirkte somit ein Verstellen der beiden Teile des Mikroskophalters 4 in X-Y- Richtung zueinander. Der Mikroskophalter 4 war dabei herkömmlich von einem Stativträger 3 gehalten, der an einem Schwenk- und/oder Drehlager 7 schwenk- und/oder drehbar gehalten war. Das Lager 7 ist nur symbolisch angedeutet. Allfällige bekannte Gewichtskompensations-Maßnahmen wie Gasdruckfedern, Seilzug-Gewichtsausgleiche usw. sind dem Fachmann bekannt und daher nicht näher dargestellt. Das Lager 7 war durch einen Mikroskop- bzw. Stativständer 2 abgestützt, der im Stativfuß 1 seine Abstützung gegen den Boden findet. Es gab im Stand der Technik selbstverständlich auch Stative, die nicht gegenüber dem Boden, sondern gegenüber der Decke abgestützt bzw. an dieser aufgehängt waren. Auch bei diesen war es jedoch üblich, die X-Y-Verschiebeeinheit im Bereich des Mikroskops anzubringen. Sinngemäß umfaßt die vorliegende Erfindung daher vergleichbare Montagearten.

Der erfindungsgemäße Aufbau gemäß Fig. 4 ist lediglich dahingehend weiterentwickelt, daß zu Gewichtskompensationszwecken der Stativträger 3c über die Längsachse des Stativständers 2a hinaus verlängert ist, um an seinem freien Ende ein Ausgleichsgewicht 8 aufzunehmen. Nicht näher dargestellt, aber dem Fachmann bekannt, sind Maßnahmen zur Justierung des Ausgleichsgewichtes 8 zum optimalen Gewichtsausgleich für das Mikroskop 6. Vorteilhafterweise muß ja erfindungsgemäß bei den Aufbauten gemäß Fig. 4 das Gewicht der X-Y-Verschiebeeinheit 5b selbst nun nicht mehr ausbalanciert werden.

Eine Alternative zur Erfindung des Aufbaus gemäß Fig. 4 führt zum Aufbau gemäß Fig. 5. Auch hier ist bei kleinstem Stativfuß 1c eine optimale Balancierung gegeben. Im Unterschied zum Aufbau gemäß Fig. 4 sind jedoch die beiden Stativträgerteile 3d zueinander schwenkbar. Zu diesem Zweck sind sie in einem Schwenktisch und/oder Drehlager 7a gelagert, das - wie nicht näher dargestellt - ein Getriebe o.dgl. aufweist, welches bewirkt, daß die beiden Teile des Stativträgers 3d voneinander abhängig jeweils in die entgegengesetzte Richtung verschwenkt werden. Das Getriebe o.dgl. ist dabei so ausgebildet, daß im statischen Zustand, d.h. wenn der Stativträger 3d nicht verschwenkt wird, er die Lastausgleichsmomente vom Mikroskop 6 zum Ausgleichsgewicht 8 überträgt. Die Verschwenkbarkeit der Stativständerteile zueinander erlaubt es, den Gesamtschwerpunkt des Statives in der Regel tief zu halten, weil in der Regel sowohl das Mikroskop als auch das Ausgleichsgewicht tiefer als der höchste Punkt des Stativständers 2 liegen.

Zur besseren Beweglichkeit des Mikroskops 6 sind bei diesem zwischen dem Mikroskophalter 4b und dem Stativträger 3d ein weiteres Schwenk- und/oder Drehlager 7b vorgesehen, das durchaus auch bei den anderen Aufbauten in den weiter beschriebenen Figuren vorgesehen sein kann.

Beim Aufbau gemäß Fig. 5 ist im Unterschied zum Aufbau gemäß Fig. 4 nicht der Stativträger 3 sondern der Stativständer 2b zur Aufnahme der X-Y-Verschiebeeinheit 5b unterbrochen. Der Ort dieser Unterbrechung wird nach konstruktiven Maßnahmen optimal gewählt. Da sich die Verschiebewege in X-Y-Richtung bei einer X-Y-Verschiebeeinheit in der Regel nur im Millimeterbereich verändern, ist die Verschiebung im Bereich des Stativständers 2 unbedenklich und führt nicht zu störenden Kippmomenten.

Im Hinblick auf die bereits erwähnte Austauschbarkeit der Erfindung in bezug auf boden- oder deckengestützte oder wandgehalterte Stative sind die Bezeichnungen Ständer und Fuß auch als Hängeträger und Basis zu verstehen. Bei einem solchen deckenmontierten Aufbau wäre selbstverständlich der Mikroskophalter und das Mikroskop auf der anderen Seite - als in den Figuren dargestellt - des Stativträgers 3 angeordnet.

Bevorzugt sind alle dargestellten X-Y-Verschiebeeinheiten auch abkoppelbar, so daß auch manuell X-Y-Verstellungen vorgenommen werden können.

Ein nicht näher dargestellter Rechner dient in Verbindung mit geeigneten Sensorelementen dazu, die jeweilige Lage des Mikroskops in bezug auf das Objekt bzw. auf einen Patienten zu erfassen und die X-Y-Befehlswerte jeweils so umzurechnen, daß die ausgeführte Verstellbewegung z.B. im X-Y-Koordinatensystem des Objektes bzw. Patienten oder im Koordinatensystem des Mikroskops durchgeführt wird; unabhängig davon, wie die Schwenklage des Mikroskops im Raum ist.

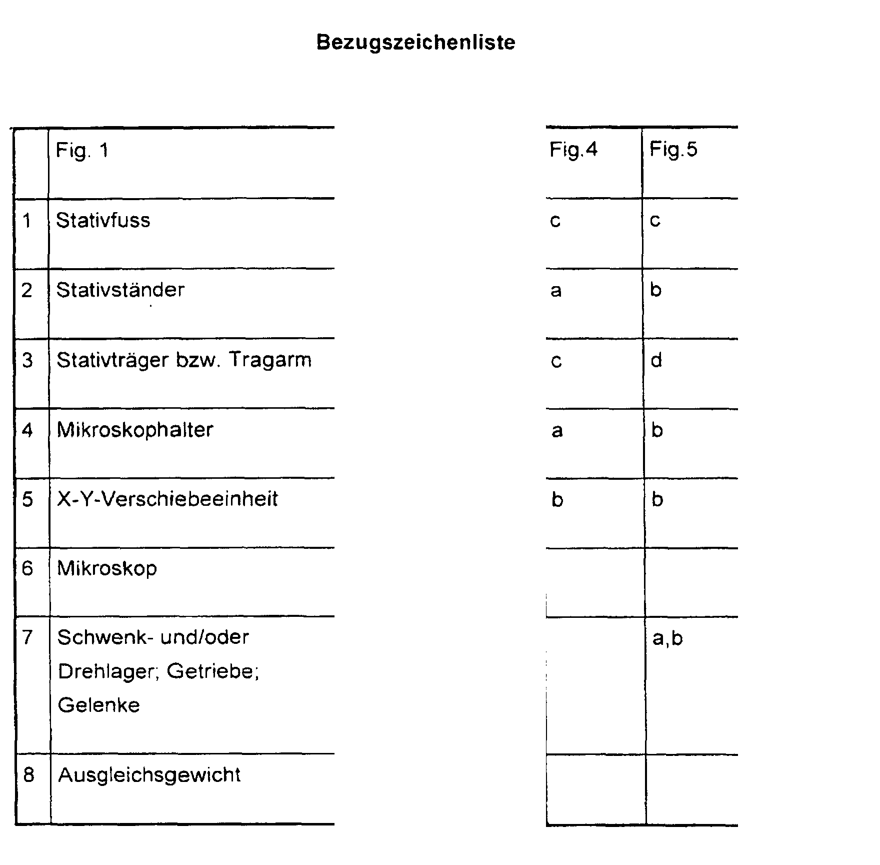

## Patentansprüche

1. Kombination einer linearen motorischen X-Y-Verschiebeeinheit mit einem Stativ eines Operationsmikroskops, letzteres Stativ mit einer Stativständersäule (2), einem ein- oder mehrteiligen Stativträgerarm (3) und einem Stativfuß (1), wobei die X-Y-Verschiebeeinheit (5) zwischen Stativständersäule (2) und dem ein- oder mehrteiligen Stativträgerarm (3) angeordnet ist und wobei der Stativträgerarm (3) zu Balancezwecken über die Längsachse der Stativständersäule (2) beidseitig erstreckt ist und ein Ausgleichsgewicht (8) trägt.

2. Kombination einer linearen motorischen X-Y-Verschiebeeinheit mit einem Stativ eines Operationsmikroskops nach Anspruch 1, **dadurch gekennzeichnet, dass** die X-Y-Verschiebeeinheit (5) neben den Einrichtungen zur linearen Verstellung in X-Y-Richtung Einrichtungen zum Schwenken des Mikroskops bzw. der Stativträgerarmteile in der X-Y-Ebene - vorzugsweise um die Längsachse der Stativständersäule - umfasst.

3. Kombination einer linearen motorischen X-Y-Verschiebeeinheit mit einem Stativ eines Operationsmikroskops nach Anspruch 1 oder 2, **dadurch gekennzeichnet, dass** die X-Y-Verschiebeeinheit (5) Elektromotore mit integrierten Inkrementalgebern umfasst.

4. Kombination einer linearen motorischen X-Y-Verschiebeeinheit mit einem Stativ eines Operationsmikroskops nach einem der vorhergehenden Ansprüche, **dadurch gekennzeichnet, dass** der Stativträgerarm (3d) über der Längsachse der Stativständersäule (2b) geteilt ist und dass beide Teile in vertikalen Ebenen zueinander schwenk- und arretierbar sind.

5. Kombination einer linearen motorischen X-Y-Verschiebeeinheit mit einem Stativ eines Operationsmikroskops nach Anspruch 4, **dadurch gekennzeichnet, dass** die beiden Teile des Stativträgerarms (3d) durch ein Getriebe (7a) miteinander verbunden sind.

6. Kombination einer linearen motorischen X-Y-Verschiebeeinheit mit einem Stativ eines Operationsmikroskops nach Anspruch 5, **dadurch gekennzeichnet, dass** das Getriebe (7a) eine - vorzugsweise elektrisch gesteuerte - Bremse für das Blockieren der beiden Teile zueinander umfasst.

7. Kombination einer linearen motorischen X-Y-Verschiebeeinheit mit einem Stativ eines Operationsmikroskops nach einem der vorhergehenden Ansprüche, **dadurch gekennzeichnet, dass** die X-Y-Verschiebeeinheit (5c) Gelenke (7c, d) umfasst, von denen wenigstens eines den Stativträgerarm (3e) unterbricht und die Teil-Stativträgerarme motorisch antreibbar in einer X-Y-Ebene verschwenkbar macht.

8. Kombination einer linearen motorischen X-Y-Verschiebeeinheit mit einem Stativ eines Operationsmikroskops nach Anspruch 1, **dadurch gekennzeichnet, dass** das Operationsmikroskop (6) und/oder der Stativträgerarm (3) gegenüber der Stativständersäule (2) schwenkbar sind und dass die X-Y-Verschiebeeinheit (5) einen Rechner umfasst, der rechnerisch allfällige willkürliche Schwenkbewegungen des Mikroskops (6) um seine eigene vertikale oder um eine vertikale Achse der Stativständersäule (2) erfasst und bei einer linearen motorischen X-Y-Verstellung berücksichtigt, sodass für den Anwender die X-Y-Richtung in Bezug auf ein Objekt bzw. auf einen Patienten immer gleich ist.

9. Kombination einer linearen motorischen X-Y-Verschiebeeinheit mit einem Stativ eines Operationsmikroskops nach einem der vorhergehenden Ansprüche, **dadurch gekennzeichnet, dass** ein Eichsystem vorgesehen ist, bei dem durch gezieltes markierungsorientiertes Positionieren des Stativaufbaus in eine Relativlage zum Patienten bzw. zum Objekt vor Arbeitsbeginn ein Reset bzw. eine Eichung durchführbar ist.

10. Kombination einer linearen motorischen X-Y-Verschiebeeinheit mit einem Stativ eines Operationsmikroskops nach einem der vorhergehenden Ansprüche, **dadurch gekennzeichnet, dass** die X-Y-Verstelleinheit (5) auch eine Z-Verstelleinheit für ein Verstellen senkrecht auf die X-Y-Ebene umfasst.

11. Kombination einer linearen motorischen X-Y-Verschiebeeinheit mit einem Stativ eines Operationsmikroskops letzteres Stativ mit einer Stativständersäule (2), einem ein- oder mehrteiligen Stativträgerarm (3) und einer Decken- oder Wandhalterung, wobei die X-Y-Verschiebeeinheit (5) zwischen Stativständersäule (2) und dem ein- oder mehrteiligen Stativträgerarm (3) angeordnet ist und wobei der Stativträgerarm (3) zu Balancezwecker über die Längsachse der Stativständersäule (2) beidseitig erstreckt ist und ein Ausgleichsgewicht (8) trägt.

## Claims

1. Combination of a linear motor-driven X-Y displacement unit with a stand of a surgical microscope, the latter stand comprising a stand column (2), a one-part or multipart stand support arm (3) and a stand foot (1), the X-Y displacement unit (5) being arranged between stand column (2) and the one-part or multipart stand support arm (3), and, for balancing purposes, the stand support arm (3) being extended on both sides beyond the longitudinal axis of the stand column (2) and carrying a counterweight (8).

2. Combination of a linear motor-driven X-Y displacement unit with a stand of a surgical microscope according to Claim 1, **characterized in that** the X-Y displacement unit (5) comprises means for pivoting the microscope or the stand support arm parts in the X-Y plane, preferably about the longitudinal axis of the stand column - in addition to the means for linear adjustment in the X-Y direction.

3. Combination of a linear motor-driven X-Y displacement unit with a stand of a surgical microscope according to Claim 1 or 2, **characterized in that** the X-Y displacement unit (5) comprises an electric motor having integrated incremental encoders.

4. Combination of a linear motor-driven X-Y displacement unit with a stand of a surgical microscope according to any of the preceding Claims, **characterized in that** the stand support arm (3d) is divided across the longitudinal axis of the stand column (2b) and that both parts are pivotable and lockable relative to one another in vertical planes.

5. Combination of a linear motor-driven X-Y displacement unit with a stand of a surgical microscope according to Claim 4, **characterized in that** the two parts of the stand support arm (3) are connected to one another by a gear (7a).

6. Combination of a linear motor-driven X-Y displacement unit with a stand of a surgical microscope according to Claim 5, **characterized in that** the gear (7a) comprises a - preferably electrically controlled - brake for blocking the two parts.

7. Combination of a linear motor-driven X-Y displacement unit with a stand of a surgical microscope according to any of the preceding Claims, **characterized in that** the X-Y displacement unit (5c) comprises joints (7c, d), at least one of which interrupts the stand support arm (3e) and makes the stand support arm parts driveable by means of a motor so as to be pivotable in an X-Y plane.

8. Combination of a linear motor-driven X-Y displacement unit with a stand of a surgical microscope according to Claim 1, **characterized in that** the surgical microscope (6) and/or the stand support arm (3) are pivotable relative to the stand column (2), and that the X-Y displacement unit (5) comprises a computer which acquires any random pivot movements of the microscope (6) about its own vertical axis or about a vertical axis of the stand column (2) and, in the case of a linear motor-driven X-Y adjustment takes into account said pivot movements so that, for the user, the X-Y direction is always the same in relation to an object or to a patient.

9. Combination of linear motor-driven X-Y displacement unit with a stand of a surgical microscope according to any of the preceding Claims, **characterized in that** a calibration system is provided in which a reset or a calibration can be carried out by specific mark-oriented positioning of the stand setup in a position relative to the patient or to the object before the beginning of work.

10. Combination of a linear motor-driven X-Y displacement unit with a stand of a surgical microscope according to any of the preceding Claims, **characterized in that** the X-Y adjustment unit (5) also comprises a Z adjustment unit for an adjustment perpendicular to the X-Y plane.

11. Combination of a linear motor-driven X-Y displacement unit with a stand of a surgical microscope, the latter stand comprising a stand column (2), a one-part or multipart stand support arm (3) and a ceiling or wall holder, the X-Y displacement unit (5) being arranged between stand column (2) and the one-part or multipart stand support arm (3), and, for balancing purposes, the stand support arm (3) being extended on both sides beyond the longitudinal axis of the stand column (2) and carrying a counterweight (8).

## Revendications

1. Combinaison d'une unité de translation en X-Y linéaire par moteur avec un statif d'un microscope d'opération, ledit statif comprenant une colonne de support de statif (2), un bras de porte-statif (3) en une ou plusieures parties et un pied de statif (1), l'unité de translation en X-Y (5) étant disposée entre la colonne de support de statif (2) et le bras de porte-statif (3) en une ou plusieures parties, dans laquelle le bras de porte-statif (3), pour le but de balance, s'étend des deux côtés au delà de l'axe longitudinale de la colonne de support de statif (2) et porte un poids de compensation (8).

2. Combinaison d'une unité de translation en X-Y linéaire par moteur avec un statif d'un microscope d'opération selon la revendication 1, **caractérisée en ce que** l'unité de translation en X-Y (5), outre les dispositifs de translation linéaire en direction X-Y, comprend des dispositifs de pivotement du microscope ou des parties du bras de porte-statif dans le plan X-Y, préférablement autour de l'axe longitudinale de la colonne de support de statif.

3. Combinaison d'une unité de translation en X-Y linéaire par moteur avec un statif d'un microscope d'opération selon la revendication 1 ou 2, **caractérisée en ce que** l'unité de translation en X-Y (5) comprend des moteurs électriques ayant des capteurs incrémentaux intégrés.

4. Combinaison d'une unité de translation en X-Y linéaire par moteur avec un statif d'un microscope d'opération selon une quelconque des revendications précédentes, **caractérisée en ce que** le bras de porte-statif (3d) est partagé au-dessus de l'axe longitudinale de la colonne de support de statif (2b), et que les deux parties sont pivotables dans des plans verticaux et sont arrêtables.

5. Combinaison d'une unité de translation en X-Y linéaire par moteur avec un statif d'un microscope d'opération selon la revendication 4, **caractérisée en ce que** les deux parties du bras de porte-statif (3d) sont connectés l'un à l'autre par un mécanisme (7a).

6. Combinaison d'une unité de translation en X-Y linéaire par moteur avec un statif d'un microscope d'opération selon la revendication 5, **caractérisée en ce que** le mécanisme (7a) comprend un frein, de préférence commandé électriquement, pour arrêter les deux parties relativement l'une à l'autre.

7. Combinaison d'une unité de translation en X-Y linéaire par moteur avec un statif d'un microscope d'opération selon une quelconque des revendications précédentes, **caractérisée en ce que** l'unité de translation en X-Y (5c) comprend des articulations (7c, d), dont au moins une interrompe le bras de porte-statif (3e), et qui rend pivotable au moins des bras partiel de porte-statif dans un plan X-Y en étant entraînés par moteur.

8. Combinaison d'une unité de translation en X-Y linéaire par moteur avec un statif d'un microscope d'opération selon la revendication 1, **caractérisée en ce que** le microscope d'opération (6) et/ou le bras de porte-statif (3) pivotent par rapport à la colonne de support de statif (2), et que l'unité de translation en X-Y (5) comprend un calculateur, qui détecte par le calcul des mouvements pivotants éventuels du microscope (6) autour de son propre axe vertical ou autour d'un axe vertical de la colonne de support de statif (2), et en tient compte à l'occasion de la translation linéaire an X-Y par moteur de manière que la direction X-Y par rapport à un objet ou par rapport à un malade est toujours la même pour l'utilisateur.

9. Combinaison d'une unité de translation en X-Y linéaire par moteur avec un statif d'un microscope d'opération selon une quelconque des revendications précédentes, **caractérisée en ce qu'**un système d'étalonnage est prévu, par lequel on peut réaliser avant le commencement du travail une remise à zéro ou un étalonnage par un positionnement approprié, orienté par marquage approprié, de la construction du statif dans une position relative au malade ou à un objet.

10. Combinaison d'une unité de translation en X-Y linéaire par moteur avec un statif d'un microscope d'opération selon une quelconque des revendications précédentes, **caractérisée en ce que** l'unité de translation en X-Y (5c) comprend aussi une unité de translation en Z pour une translation perpendiculairement au plan X-Y.

11. Combinaison d'une unité de translation en X-Y linéaire par moteur avec un statif d'un microscope d'opération, ledit statif comprenant une colonne de support de statif (2), un bras de porte-statif (3) en une ou plusieurs parties et un dispositif d'attache au plafond ou à la paroi, l'unité de translation en X-Y (5) étant disposée entre la colonne de support de statif (2) et le bras de porte-statif (3) en une ou plusieurs parties, dans laquelle le bras de porte-statif (3), pour le but de balance, s'étend des deux côtés au delà de l'axe longitudinale de la colonne de support de statif (2) et porte un poids de compensation (8).
